# EUROPEAN PATENT SPECIFICATION

(11) **EP 3 090 747 B1**
(45) Date of publication and mention of the grant of the patent: **09.12.2020**
(21) Application number: 16170271.7
(22) Date of filing: 07.10.2011
(51) Int. Cl.: A61K 31/554

(54) **CONTROLLED-RELEASE FORMULATIONS OF QUETIAPINE**
QUETIAPIN-FORMULIERUNGEN MIT GESTEUERTER FREISETZUNG
FORMULATIONS DE QUÉTIAPINE À LIBÉRATION CONTRÔLÉE

(30) Priority: 08.10.2010 TR 201008261
(43) Date of publication of application: 09.11.2016
(62) Divisional of application: 11184366.0
(73) Proprietor: SANOVEL ILAC SANAYI VE TICARET A.S., 34460 Sariyer/Istanbul (TR)
(72) Inventor: CIFTER, Ümit, 34398 Istanbul (TR); TÜRKYILMAZ, Ali, 34398 Istanbul (TR); YILDIRIM, Ediz, 34398 Istanbul (TR); ERDEM, Yelda, 34398 Istanbul (TR); ERGENEKON, Ercan, 34398 Istanbul (TR); ÖNER, Levent, 34398 Istanbul (TR)
(74) Representative: Sonnenhauser, Thomas Martin

(56) References cited:
- EP-A2- 2 153 834
- WO-A2-2010/089259
- US-A1- 2007 244 093
- US-A1- 2009 220 593
- US-A1- 2009 264 408
- "AQUALON Ethylcellulose - Product Data", INTERNET CITATION, 2008, XP002620408, Retrieved from the Internet: URL:http://www.signetchem.com/downloads/da tasheets/Ashland-Aqualon/PDS-Aqualon-EC-sp ecifications.pdf [retrieved on 2011-02-03]
- ROWE ET AL: "The effect of the molecular weight of ethyl cellulose on the drug release properties of mixed films of ethyl cellulose and hydroxypropylmethylcellulose", INTERNATIONAL JOURNAL OF PHARMACEUTICS, ELSEVIER BV, NL, vol. 29, no. 1, 1 March 1986 (1986-03-01), pages 37-41, XP025502228, ISSN: 0378-5173, DOI: DOI:10.1016/0378-5173(86)90197-3 [retrieved on 1986-03-01]
- P.K. KATIKANEMI ET AL.: "Ethylcellulose matrix controlled release tabletds of a water-soluble drug", INTERNATIONAL JOURNAL OF PHARMACEUTICS, ELSEVIER BV, NL, vol. 123, 1995, pages 119-125, XP002620409,

## Description

### Field of Invention

The present invention relates to a novel pharmaceutical formulation as defined in the claims comprising quetiapine fumarate salt, or a solvate, or a polymorph thereof. The invention more particularly relates to a controlled-release formulation of quetiapine as defined in the claims, uniformly dispersed in a matrix tablet and used as a single daily dose.

### Background of Invention

Quetiapine fumarate, a dibenzothiazepine derivative, is an atypical antipsychotic. It ameliorates the negative and positive symptoms of schizophrenia, without giving rise to extrapiramidal side effects. Similar to clozapine, quetiapine shows moderate dopamine D2-receptor antagonist and potent 5-HT2-receptor antagonist effects. The chemical designation of quetiapine fumarate is 2-[2-(4-dibenzo[b,f][1,4]thiazepine-11-yl-1-piperazinyl)ethoxy] ethanol fumarate, with the following chemical structure of Formula I.

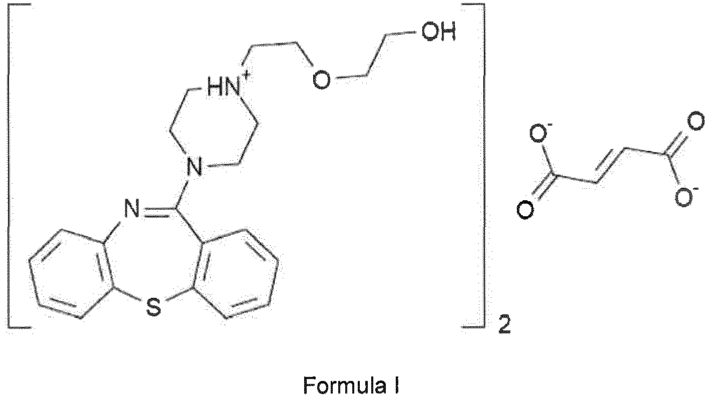

Extended release tablets of quetiapine is marketed under the name Seroquel XR® and is administered orally once in a day. These tablets comprise 50 mg, 150 mg, 200 mg, 300 mg or 400 mg quetiapine fumarate as an active agent.

The quetiapine molecule was disclosed in the applications EP0240228 and US4879288. EP282236 discloses a process for the preparation of a quetiapine molecule.

The patent EP2153834 discloses an extended release multiparticulate formulation comprising quetiapine or a pharmaceutical acceptable salt thereof and an excipient and hydrophobic release controlling agent.

US20080287418 discloses an extended release formulation comprising 9.6 - 10.4% quetiapine, 30% hydroxypropyl methyl cellulose, and 7.2% sodium citrate dihydrate.

WO2010012490 discloses a controlled release formulation comprising quetiapine and a non-gelling carrageenan.

WO9745124 discloses a formulation comprising gelling agent, quetiapine, and one or more excipients. There are mentioned on various polymers in that patent for use as a gelling agent. Hydroxypropyl methyl cellulose, hydroxypropyl cellulose, hydroxymethyl cellulose, hydroxyethyl cellulose, carbomer, sodium carboxymethyl cellulose, polyvinylpyrrolidone, ethyl cellulose, methyl cellulose, carboxymethyl cellulose are specified in that document as gelling agents.

It is a desired feature to obtain an active pharmaceutical agent in a modified-release form in the treatment of many diseases. What is desired with a modified release is to reach desired plasma levels of an active agent of interest throughout a determined period of time, and to provide the drug release at a uniform and constant rate. Thus, the drug administration frequency can be lowered.

There are various difficulties associated with developing a controlled-release formulation. One of the problems encountered is dose damping, by which a drug obtained is released too rapidly. Obtaining intended solubility profiles is also difficult, i.e. it is difficult to control the release rate. For this reason, there may occur variations in the plasma concentrations of active agents, which may lead to toxicity. In addition, there are also daily alterations possible for the active agent in plasma.

There are various formulations available, which have been developed with quetiapine fumarate. There are various problems, however, associated with quetiapine fumarate formulations.

Quetiapine fumarate has a low dissolution rate. Producing controlled-release tablets of quetiapine fumarate and similar active agents having low solubility rates, and providing desired release profiles in these tablets are quite difficult. Generating release amounts at desired intervals bears vital importance with respect to patients. Obtaining a desired release profile depends on the convenience of the excipients to be selected.

Quetiapine fumarate is also a low-density substance. Therefore the tablet production process of quetiapine fumarate is quite difficult. Tablets produced are prone to erosion and disintegration.

Quetiapine fumarate is to be stored below temperatures of 30°C under normal conditions. Stability-related problems are encountered in temperatures exceeding this point. Failing to prepare a formulation with convenient excipients leads to stability problems.

Accordingly, there is a need towards modified-release formulations of quetiapine or a pharmaceutically-acceptable salt thereof, which would overcome the problems referred to hereinabove, and would provide drug plasma concentrations equivalent or better than those of the currently-used immediate-release tablet formulations. The formulation according to the present invention provides a novel modified-release formulation, not disclosed in the relevant art yet.

The modified-release formulation according to the present invention both provides plasma levels comparable to the immediate-release form thereof, and gives an advantageous feature of administering the active drug less frequently, e.g. once or twice a day.

Due to the reasons specified above, there is a need towards a stable pharmaceutical formulation of quetiapine or pharmaceutically acceptable salts, solvates, or hydrates thereof, providing a proper content uniformity and a desired release profile.

### Object and Brief Description of Invention

The present invention relates to a controlled-release formulation of quetiapine, eliminating all aforesaid problems and brining additional advantages to the relevant prior art.

Accordingly, the main object of the present invention is to obtain a controlled-release formulation comprising quetiapine fumarate as defined in the claims, which is stable and provides a desired release profile.

Another object of the present invention is to obtain a controlled-release formulation comprising quetiapine fumarate as defined in the claims with a desired dissolution rate thanks to employing proper excipients.

A further object of the present invention is to ensure a uniform distribution of active agent and excipients in said formulations, so that a tablet form thereof will not be prone to erosion.

A controlled-release pharmaceutical formulation comprising quetiapine fumarate as defined in the claims has been developed to carry out all objects, referred to above and to emerge from the following detailed description.

The invention thus refers to a pharmaceutical formulation in the form of a tablet comprising a core and an optional coating comprising quetiapine fumarate salt, or a solvate, or a polymorph thereof, characterized in that the core of said formulation comprises a non-gelling ethyl cellulose with an ethoxyl content of 48 - 49.5% and a viscosity of 18 to 24 mPa.s, wherein the proportion by weight of ethyl cellulose to the total weight of formulation is between 20 to 40%, wherein said quetiapine is in the form of its fumarate salt, and wherein the weight ratio of quetiapine to ethyl cellulose is between 0.1 to 10.

Thanks to the present invention developed, a stable controlled-release formulation is surprisingly obtained thanks to making use of non-gelling ethyl cellulose with an ethoxyl content of 48 - 49.5% in which quetiapine is uniformly distributed, thereby solving said problems and allowing to achieve the desired release profile. Thanks to the formulation developed, the risk of dose dumping is eliminated and a controlled-release formulation is obtained by using a specific non-gelling ethyl cellulose.

According to a preferred embodiment of the present invention, as much as 35%, preferably as much as 30% of quetiapine fumarate salt is released in 2 hours; 30 to 65%, preferably 35 to 55% of quetiapine fumarate salt is released in 4 hours; and a minimum of 80% of quetiapine or a pharmaceutically acceptable salt thereof is released in 16 hours.

According to the present invention, the weight ratio of quetiapine to ethyl cellulose is between 0.1 to 10, preferably 1 to 4, and more preferably 1.5 to 3.

According to a preferred embodiment of the present invention, the proportion by weight of ethyl cellulose to the total weight of formulation is between 20 to 40%.

According to another preferred embodiment of the present invention, excipients used comprise at least one or a mixture of fillers, buffering agents, glidants, lubricants.

According to a preferred embodiment of the present invention, said filler is at least one of microcrystalline cellulose and lactose monohydrate, or a properly-proportioned mixture thereof.

According to a preferred embodiment of the present invention, said buffering agent is sodium citrate dihydrate. With using sodium citrate dihydrate in the formulation, the desired release profile can be obtained independent from the pH of the medium.

According to another preferred embodiment of the present invention, the proportion by weight of ethyl cellulose to sodium citrate dihydrate is between 0.2 to 60, preferably 1 to 50, and more preferably between 2 to 40.

According to a preferred embodiment of the present invention, said glidant is colloidal silicon dioxide.

According to a preferred embodiment of the present invention, magnesium stearate is used as a lubricant.

According to a preferred embodiment of the present invention, said tablet is an uncoated tablet.

A further preferred embodiment according to the present invention provides a method for preparing a pharmaceutical formulation according to the invention, this method comprising the steps of
a. mixing quetiapine, filler and controlled-release agent together,
b. forming wet granulation with an alcohol-water mixture,
c. wet milling and drying,
d. sieving the granules,
e. adding lubricant-glidant into the sieved powder mixture obtained with granular structure and mixing the latter,
f. compressing into tables, and
g. coating the tablets.

According to another preferred embodiment of to the present invention, said pharmaceutical formulation is consisting of
a. core
   a. quetiapine fumarate at 33 to 70% by weight
   b. non-gelling ethyl cellulose as defined in the claims at 20 to 40% by weight
   c. sodium citrate dihydrate at 2 to 30% by weight
   d. lactose monohydrate at 3.0 to 50% by weight
   e. microcrystalline cellulose at 4.0 to 60% by weight
   f. colloidal silicone dioxide at 0.2 to 5.0% by weight
   g. magnesium stearate at 0.1 to 5.0% by weight
b. coating
   a. hydroxymethylpropyl cellulose or polyvinyl alcohol at 1.0 to 5.0% by weight.

### Detailed Description of Invention

### Example

| **Content** | **Amount (%) (w/w)** |
|---|---|
| quetiapine fumarate | 51.2 |
| ethyl cellulose (ethoxyl content 48 - 49.5%) | 22.0 |
| sodium citrate dihydrate | 3.0 |
| lactose monohydrate | 4.6 |
| microcrystalline cellulose | 16.2 |
| colloidal silicone dioxide | 0.6 |
| magnesium stearate | 2.4 |

Ethyl celluloses, commercially available under the trademark Aqualon®, used in the present invention has four different types according their ethoxyl content.

| **Type** | **Ethoxyl content (%)** |
|---|---|
| K type* | 45 - 47,2 |
| N type | 48 - 49,5 |
| T type* | 49,6 - 51,5 |
| X type* | 50,5 - 52,5 |

| | |
|---|---|
| * not claimed (see the 2002-document specifying physical and chemical specifications of the product Aqualon ® of the company Herkules, p. 3.) | |

Ehtyl celluloses with type K, N, T, X, as indicated in the table, have different ethoxyl contents and show characteristic features in line with content rates. The type K, among these 4 types of ethyl cellulose gelates in organic solvents (toluene, xylene, butyl acetate, carbon tetrachloride), but the types N, T, and X does not gelate. With the invention made, at least one or a properly-proportioned mixture of the type N, T, and X ethyl celluloses are used.

The N type and/or T type ethyl cellulose used in the formulation does not dissolve in water and becomes not gelated. Specifically-selected excipients are used together with the N type and/or T type ethyl cellulose in the formulation. Sodium citrate dihydrate, among these excipients, generates a proper pH medium around the ethyl cellulose matrix and facilitates its solubility. Thanks to this feature, the release profile of the formulation is brought to the desired level independently from the pH of the medium.

Thanks to the present invention developed, a stable controlled-release formulation is surprisingly obtained thanks to making use of ethyl cellulose with an ethoxyl content of 48 - 49.5% in which quetiapine fumarate is uniformly distributed, thereby solving said problems and allowing to achieve the desired release profile. Thanks to the formulation developed, the risk of dose damping is eliminated and a controlled-release formulation is obtained by using non-gelling ethyl cellulose. The core of said formulation comprises a non-gelling ethyl cellulose with an ethoxyl content of 48 - 49,5 %.

The formulation is made as follows. Quetiapine fumarate, filling agent, and controlled-release agent are mixed together. Then this mixture is subjected to a wet milling process, together with an alcohol-water mixture. Then, the granules obtained by wet milling and then dried are sieved. Thereafter, lubricant-glidant is added to the sieved powder mixture with a granular form and the resulting mixture is mixed again. Finally, the mixture obtained is compressed into tablets and the tablets obtained are coated.

N type ethyl cellulose has the following viscosity ranges.

| **Type** | **Viscosity range (mPa.s)** |
|---|---|
| N7 Pharm* | 6 - 8 |
| N10 Pharm* | 8 - 11 |
| N14 Pharm* | 12 - 16 |
| N22 Pharm | 18 - 24 |
| N50 Pharm* | 40 - 52 |
| N100 Pharm* | 80 - 105 |
| T 10 Pharm* | 8 - 11 |
| T 50 Pharm* | 40 - 52 |

| | |
|---|---|
| * not claimed | |

It is further possible to use the following additional excipients in the formulation.

Suitable binders include, but are not restricted to at least one or a mixture of polyvinylpyrrolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to at least one or a mixture of colloidal silicone dioxide, talk, aluminum silicate.

Suitable lubricants include, but are not restricted to at least one or a mixture of sodium stearil fumarate, magnesium stearate, polyethylene glycol, stearic acid, metal stearates, boric acid, sodium chloride benzoate and acetate, sodium or magnesium lauryl sulfate.

Suitable colorants include, but are not restricted to at least one or a mixture of food, drug, and cosmetic (FD&C) dyes (FD&C blue, FD&C green, FD&C red, FD&C yellow, FD&C lake), ponceau, indigo Drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

The invention also refers to the pharmaceutical formulations of the invention for use in a method of treatment of bipolar disease, obsessive-compulsive disorder and schizophrenia, mania, depression, dementia, agitation disorders.

## Claims

1. A pharmaceutical formulation in the form of a tablet comprising a core and an optional coating, the core comprising quetiapine fumarate salt, or a solvate, or a polymorph thereof, **characterized in that** the core of said formulation comprises a non-gelling ethyl cellulose with an ethoxyl content of 48 - 49.5% and a viscosity of 18 to 24 mPa.s,
wherein the proportion by weight of ethyl cellulose to the total weight of formulation is between 20 to 40%, wherein said quetiapine is in the form of its fumarate salt, and wherein the weight ratio of quetiapine to ethyl cellulose is between 0.1 to 10.

2. The controlled-release formulation according to claim 1, wherein the core of said formulation comprises a non-gelling ethyl cellulose with an ethoxyl content of 48 - 49.5%.

3. The controlled-release formulation according to any of the preceding claims, wherein the weight ratio of quetiapine to ethyl cellulose is between 1 to 4, and preferably 1.5 to 3.

4. The controlled-release formulation according to any of the preceding claims, further containing excipients, comprising at least one or a mixture of fillers, buffering agents, glidants, lubricants.

5. The pharmaceutical formulation according to any of the preceding claims, wherein said filler is at least one of microcrystalline cellulose and lactose monohydrate, or a properly-proportioned mixture thereof.

6. The pharmaceutical formulation according to any of the preceding claims, wherein said buffering agent is sodium citrate dihydrate.

7. The controlled-release formulation according to any of the preceding claims, wherein the weight ratio of ethyl cellulose to sodium citrate dihydrate is between 0.2 to 60, preferably 1 to 50, and more preferably between 2 to 40.

8. The pharmaceutical formulation according to any of the preceding claims, wherein said glidant is colloidal silicone dioxide.

9. The pharmaceutical formulation according to any of the preceding claims, wherein said lubricant is magnesium stearate.

10. A method for preparing a pharmaceutical formulation according to any of the preceding claims, comprising the steps of
a. mixing quetiapine, filler and controlled-release agent together,
b. forming wet granulation with an alcohol-water mixture,
c. wet milling and drying,
d. sieving the granules,
e. adding lubricant-glidant into the sieved powder mixture obtained with granular structure and mixing the latter,
f. compressing into tablets, and
g. coating the tablets.

11. A pharmaceutical formulation according to any of the preceding claims for use in preventing or treating bipolar disease, obsessive-compulsive disorder and schizophrenia, mania, depression, dementia, agitation disorders in mammalians and particularly in humans.

12. A tablet obtainable or obtained by the method of claim 10.

## Patentansprüche

1. Eine pharmazeutische Formulierung in der Form einer Tablette, die einen Kern und eine optionale Beschichtung umfasst, wobei der Kern Quetiapinfumarat-Salz, oder ein Solvat, oder ein Polymorph davon umfasst, **dadurch gekennzeichnet, dass** der Kern der Formulierung eine nicht-gelierende Ethylcellulose mit einem Ethoxylgehalt von 48 - 49,5% und einer Viskosität von 18 bis 24 mPa.s umfasst, wobei der Gewichtsanteil von Ethylcellulose am Gesamtgewicht der Formulierung zwischen 20 und 40% liegt, wobei das Quetiapin in der Form seines Fumarat-Salzes vorliegt, und wobei das Gewichtsverhältnis von Quetiapin zu Ethylcellulose zwischen 0,1 und 10 beträgt.

2. Die Formulierung mit kontrollierter Freisetzung nach Anspruch 1, wobei der Kern der Formulierung eine nicht-gelierende Ethylcellulose mit einem Ethoxylgehalt von 48 - 49,5% umfasst.

3. Die Formulierung mit kontrollierter Freisetzung nach einem der vorangehenden Ansprüche, wobei das Gewichtsverhältnis von Quetiapin zu Ethylcellulose zwischen 1 und 4, und vorzugsweise 1,5 bis 3 beträgt.

4. Die Formulierung mit kontrollierter Freisetzung nach einem der vorangehenden Ansprüche, die des Weiteren Hilfsstoffe enthält, die mindestens eines von oder ein Gemisch an Füllmitteln, Pufferagenzien, Gleitmitteln, Schmiermitteln umfassen.

5. Die pharmazeutische Formulierung nach einem der vorangehenden Ansprüche, wobei das Füllmittel mindestens eines von mikrokristalliner Cellulose und Laktosemonohydrat, oder ein angemessen-proportioniertes Gemisch davon ist.

6. Die pharmazeutische Formulierung nach einem der vorangehenden Ansprüche, wobei das Pufferagens Natriumcitratdihydrat ist.

7. Die Formulierung mit kontrollierter Freisetzung nach einem der vorangehenden Ansprüche, wobei das Gewichtsverhältnis von Ethylcellulose zu Natriumcitratdihydrat zwischen 0,2 bis 60, vorzugsweise 1 bis 50 und mehr bevorzugt zwischen 2 bis 40 beträgt.

8. Die pharmazeutische Formulierung nach einem der vorangehenden Ansprüche, wobei das Gleitmittel kolloidales Siliziumdioxid ist.

9. Die pharmazeutische Formulierung nach einem der vorangehenden Ansprüche, wobei das Schmiermittel Magnesiumstearat ist.

10. Ein Verfahren zur Herstellung einer pharmazeutischen Formulierung nach einem der vorangehenden Ansprüche, umfassend die Schritte
a. Mischen von Quetiapin, Füllmittel und Agens zur kontrollierten Freisetzung,
b. Bilden von Feuchtgranulat mit einem Alkohol-Wasser-Gemisch,
c. Nassvermahlung und Trocknen,
d. Sieben der Granula,
e. Hinzufügen von Schmiermittel-Gleitmittel in das gesiebte Pulvergemisch, das mit körniger Struktur erhalten wurde und Mischen des Letzteren,
f. Verpressen zu Tabletten, und
g. Beschichten der Tabletten.

11. Eine pharmazeutische Formulierung gemäß einem der vorangehenden Ansprüche zur Anwendung bei der Vorbeugung oder der Behandlung von bipolarer Störung, Zwangsstörung und Schizophrenie, Manie, Depression, Demenz, Agitationsstörungen bei Säugetieren und insbesondere bei Menschen.

12. Eine Tablette, erhältlich oder erhalten nach dem Verfahren von Anspruch 10.

## Revendications

1. Formulation pharmaceutique sous la forme d'un comprimé comprenant un cœur et un enrobage facultatif, dans lequel le cœur comprend du sel de fumarate de quétiapine, ou un solvate, ou un polymorphe de celui-ci, **caractérisée en ce que** le cœur de ladite formulation comprend une éthylcellulose non gélifiante avec une teneur en éthoxyle de 48 à 49,5 % et une viscosité de 18 à 24 mPa.s,
dans laquelle la proportion en poids d'éthylcellulose au poids total de formulation est comprise entre 20 et 40 %, dans laquelle ladite quétiapine est sous la forme de son sel de fumarate, et dans laquelle le rapport en poids de la quétiapine à l'éthylcellulose est compris entre 0,1 et 10.

2. Formulation à libération contrôlée selon la revendication 1, dans laquelle le cœur de ladite formulation comprend une éthylcellulose non gélifiante avec une teneur en éthoxyle de 48 à 49,5 %.

3. Formulation à libération contrôlée selon l'une quelconque des revendications précédentes, dans laquelle le rapport en poids de la quétiapine à l'éthylcellulose est compris entre 1 et 4, et de préférence entre 1,5 et 3.

4. Formulation à libération contrôlée selon l'une quelconque des revendications précédentes, contenant en outre des excipients dont au moins une charge ou un mélange de charges, des agents tampons, des agents de glissement, des lubrifiants.

5. Formulation pharmaceutique selon l'une quelconque des revendications précédentes, dans laquelle ladite charge est au moins une charge parmi une cellulose microcristalline et un lactose monohydraté, ou un mélange correctement proportionné de ceux-ci.

6. Formulation pharmaceutique selon l'une quelconque des revendications précédentes, dans laquelle ledit agent tampon est le citrate de sodium dihydraté.

7. Formulation à libération contrôlée selon l'une quelconque des revendications précédentes, dans laquelle le rapport en poids de l'éthylcellulose au citrate de sodium dihydraté est compris entre 0,2 et 60, de préférence entre 1 et 50, et plus préférablement entre 2 et 40.

8. Formulation pharmaceutique selon l'une quelconque des revendications précédentes, dans laquelle ledit agent de glissement est le dioxyde de silicium colloïdal.

9. Formulation pharmaceutique selon l'une quelconque des revendications précédentes, dans laquelle ledit lubrifiant est le stéarate de magnésium.

10. Procédé de préparation d'une formulation pharmaceutique selon l'une quelconque des revendications précédentes, comprenant les étapes suivantes :
a. mélange de quétiapine, d'une charge et d'un agent de libération contrôlée,
b. formation d'une granulation par voie humide à l'aide d'un mélange alcooleau,
c. broyage à l'état humide et séchage,
d. tamisage des granulés,
e. ajout de lubrifiant-agent de glissement au mélange pulvérulent tamisé obtenu ayant une structure granulaire et homogénéisation de ce dernier,
f. compression pour obtenir des comprimés, et
g. enrobage des comprimés.

11. Formulation pharmaceutique selon l'une quelconque des revendications précédentes destinée à être utilisée pour prévenir ou traiter les troubles bipolaires, les troubles obsessionnels compulsifs et la schizophrénie, la manie, la dépression, la démence, un trouble du comportement de type agitation chez les mammifères et en particulier chez l'homme.

12. Comprimé pouvant être obtenu ou obtenu par le procédé de la revendication 10.
